Europäisches Patentamt

European Patent Office

Office européen des brevets

(11) Publication number: **0 363 533**
**A1**

# EUROPEAN PATENT APPLICATION

(21) Application number: 88309547.3

(22) Date of filing: 12.10.88

(51) Int. Cl.⁵: **A61F 13/20**

(43) Date of publication of application:
18.04.90 Bulletin 90/16

(84) Designated Contracting States:
**DE FR GB**

(71) Applicant: Iwahashi, Tsugio
103, Ippongi-cho 3 chome
Handa-shi Aichi-ken(JP)

Applicant: Chisso Corporation
6-32, Nakanoshima 3-chome Kita-ku
Osaka-shi Osaka-fu(JP)

(72) Inventor: Tsugio, Iwahashi
103, Ippongi-cho, 3-chome, Handa-shi
Aichi-ken(JP)
Inventor: Masahiko, Matsuno c/o Chisso
Corporation
6-32 Nakanoshima 3-chome
Kita-ku, Osaka-shi, Osaka-fu(JP)

(74) Representative: Lamb, John Baxter et al
MARKS & CLERK 57/60 Lincoln's Inn Fields
London WC2A 3LS(GB)

(54) **Applicator.**

(57) An applicator used for applying liquefied medicine to an affected part of the human body or for artificial insemination of the fruit trees includes a bar (1) and a group of fibers (2) affixed to one or both ends of the bar (1). Each fiber includes a heat bondable bicomponent fiber having a meltable layer (4) on the surface. The heat bondable bicomponent fibers are heat-treated and combined with one another so that the fibers are prevented from being frayed. Each heat bondable bicomponent fiber consists of a first component composed of a polymer having fiber-forming property and a second component composed of a polymer having a fiber-forming property and a melting point at least 20°C lower than that of the first component. The first and second components are arranged into a parallel or sheathed configuration such that a ratio of an arc portion of the cross sectional circumference of the second component to cross sectional circumference of the first component ranges from 50% to 100%.

Fig. 1

# APPLICATOR

This invention relates to an applicator comprising a bar and a cotton-form group of fibers affixed to either or both ends of the bar and used for applying liquefied medicine to an affected part of a human body or for artificial insemination of fruit trees.

An applicator used for the above-mentioned purposes generally comprises a bar and a group of fibers affixed to one or both ends of the bar. A long use of such an applicator causes the fibers to be frayed from the surface of the group of fibers, thereby reducing the usability of the applicator. For the prevention of reducing the usability of the applicator, the group of fibers is conventionally immersed in a solution of paste consisting of a water soluble high polymer compound and the like in a manufacturing step so that the paste is affixed to the surfaces of fibers, thereby securing the clinging of the fibers.

However, since there are many cases where the group of fibers is soaked with a water soluble medicine or where the human wet skin is rubbed with the applicator, the water soluble paste affixed to the surfaces of fibers is gradually dissolved. Consequently, the effect of the paste for preventing the fibers from being frayed from the fiber surfaces does not last long.

Therefore, an object of the present invention is to provide an applicator which has fine water proof and endurability.

To attain the above-described object, the applicator in accordance with the present invention comprises a bar and a group of fibers affixed to either or both of the ends of the bar. A part of the fibers each consists of a heat bondable bicomponent fiber having a meltable layer. Heat is applied to the heat bondable bicomponent fiber, which are frayed.

The heat bondable bicomponent fiber consists of a first component including a polymer having fiber forming property and a second component including a polymer having fiber forming property and a melting point at least 20° C lower than that of the first component. The first and second components are combined with each other to be arranged in parallel with each other, or the second component is combined with the first component so as to be sheathed by the first component so that the ratio of an arc portion of the cross sectional circumference of the second component to the cross sectional circumference of the first component ranges from 50% to 100%.

Additionally, the group of fibers may not consist of only the heat bondable bicomponent fibers. Alternatively, each heat bondable bicomponent fiber may compose 10 weight percentage of the group of fibers with the remainder composed of synthetic or natural fibers.

In the manufacturing of the above-described applicators, the heating treatment for combining the heat bondable bicomponent fibers with one another may be executed either after or before the group of fibers is affixed to the end or ends of the applicator.

According to the applicator described above at least a part of the fibers composing the group consists of heat bondable bicomponent fibers. Since the heat bondable bicomponent fibers are melted and combined with one another by way of heating treatment, the heat bondable bicomponent fibers are not dissolved even when the fiber group of the applicator is subjected to liquid.

When the melting point of the heat bondable bicomponent composing the second component is rendered 20° C lower than that of the first component, the first component is not melted although the second component is in the melting state of condition owing to the heating treatment. As a result, the temperature control at a manufacturing step of combining the fibers with one another by the heating treatment may be performed with ease.

Furthermore, in the case where the ratio of an arc portion of the cross sectional circumference of the second component to that of the cross sectional circumference of the first component ranges from 50% to 100%, the heat bondable bicomponent fibers may be efficiently melted and combined with one another, and interfacial separation between the first and second components may be prevented.

In the case where a part of the fibers is composed of the heat bondable bicomponent fibers the hardness of the fiber group may be controlled depending on the heat bondable bicomponent fiber content ratio, thereby providing applicators having suitable characteristics in accordance with objects of usage thereof.

Embodiments of the invention will now be described, by way of example, with reference to the accompanying drawings, in which:

Fig. 1 is a side view of the applicator of a first embodiment in accordance with the invention;

Fig. 2 is a transverse sectional view of the heat bondable bicomponent fiber employed in the applicator:

Fig. 3 is a transverse sectional view of the heat bondable bicomponent fiber of a modified form; and

Fig. 4 is a view similar to Fig. 1 showing the applicator of a second embodiment.

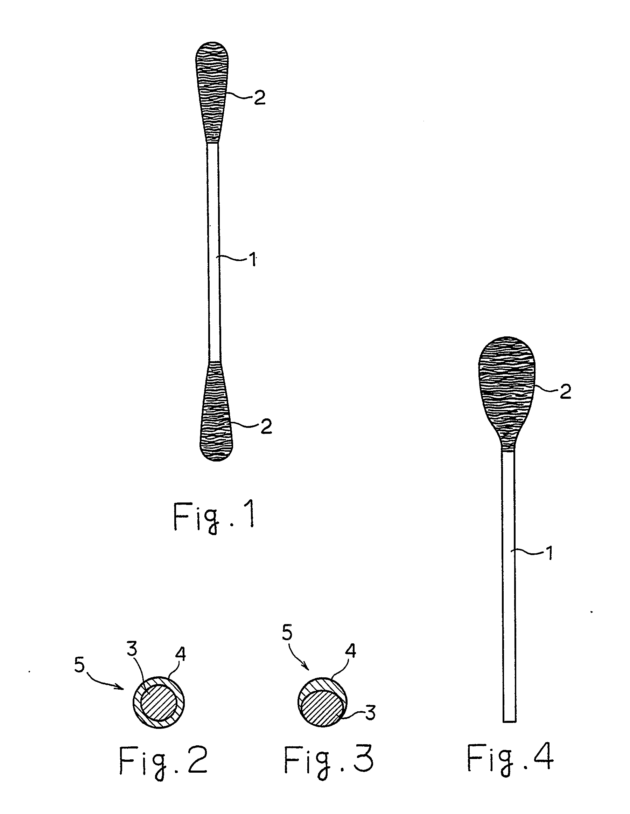

The applicator of a first embodiment will now be described with reference to Figs. 1 and 2.

Referring to Fig. 1 schematically illustrating the applicator, an applicator bar 1 is formed from plastics, wood, or paper. Two groups of fibers are affixed to both ends of the applicator bar 1, respectively to be each shaped into a form of cotton. Prior to the affixing of the fiber groups, adhesive (not shown) is applied to the ends of the applicator bar 1 so that adherence of the fiber groups 2 to the bar 1 may be enhanced.

Each fiber group 2 is composed of two kinds of fibers. One of them is a heat bondable bicomponent fiber. Fig. 2 illustrates an enlarged transverse sectional view of the heat bondable bicomponent fiber. A meltable layer 4 which melts by application of heet thereto is provided round the surface of a core 3. The core 3 is composed of a first component consisting of a polymer having fiber forming property. The meltable layer 4 is composed of a second component consisting of a polymer having fiber forming property and a melting point at least 20°C lower than that of the first component. As a combination of the polymers having fiber forming property, for example, polypropylene and high density polyethylene are used as the first and second components, respectively. The polymers having fiber forming property are not limited to this combination. Other combinations are high density polyethylene as the first component and low density polyethylene as the second component, polypropylene and ethylene vinylacetate, polyester and polypropylene, and high density polyethylene and ethylene vinylacetate. Should the second component have a melting point at least 20°C lower than that of the first component, further other combinations of the polymers having fiber forming property may be employed. Additionally, two or more kinds of polymers having fiber forming property may be employed for each of the first and second components.

The heat bondable bicomponent fiber 5 described above may be obtained by the use of a bicomponent type nozzle of the multilayer configuration. The first component is discharged from the central hole and simultaneously, the second component is discharged from the outer holes, whereby spinning the fibers. A bicomponent type nozzle of the parallel configuration may be used instead of the multilayer type. In the case of the bicomponent type nozzle of the parallel configuration, the meltable layer 4 (the second component) having a generally crescent-shaped cross section is formed round the core 3 (the first component) having an approximately circular cross section, as shown in Fig. 3. When the first and second components are combined with each other into a parallel arrangement, it is desirable that the ratio of an arc portion of the cross sectional circumference of the second component to the cross sectional circumference of the first component take the value ranging from 50% to 100%. Where the ratio takes the value smaller than the range, the degree of the melting of the fibers is rendered low, and an interfacial stripping is likely to occur between the first and second components. In the case of the sheathed arrangement, the ratio of the arc portion of the cross sectional circumference of the second component to the cross sectional circumference of the first component takes the value of 100%, as shown in Fig. 2. Therefore, the required value of the ratio ranges from 50% to 100%.

In the embodiment, a synthetic fiber having a melting point higher than that of the first component such as polyester fiber or a natural fiber having no melting characteristic is employed as the remainder of the fiber group 2. The ratio of the heat bondable bicomponent fiber to the fiber group 2 depends upon the components composing the heat bondable bicomponent fiber 5. In the embodiment, a most desirable result can be attained when the ratio of the heat bondable bicomponent fiber 5 to the fiber group 2 takes the value of 10 weight percentage of the fiber group 2 or above. When the heat bondable bicomponent fiber 5 takes the value smaller than 10 weight percentage, a desirable melting effect cannot be obtained from the heat bondable bicomponent fiber 5 and accordingly, a desirable effect to prevent the fraying of the fiber group 2 cannot be obtained.

In manufacturing applicators employing the above-described heat bodable bicomponent fiber 5 and plastic axis, both ends of the bar are roughed with a wire brush beforehand so that the fiber group 2 is easily affixed to each end of the bar 1. While, in the case that the bar 1 is formed from wood or paper, water-proof adhesive is applied to the ends of the bar 1 beforehand.

Subsequently, the heat bondable bicomponent fiber 5 and natural fiber or the like are slivered and combined with each other into a cotton form in a method well known in the art. The fibers are then affixed to both ends of the bar 1 with a suitable winding machine, thereby forming desirable fiber groups 2 on both ends of the bar 1, respectively.

The applicator is then placed, for a predetermined period of time, in an atmosphere, the temperature of which ranges between the melting point of the second component composing the meltable layer 4 of the heat bondable bicomponent fiber 5 and the melting point of the first component composing the core 3 of the heat bondable bicomponent fiber 5, thereby melting at least the surface of the meltable layer 4 temporally. A heating furnace or other heating apparatus are used to provide the above-mentioned atmosphere. The heat bondable bicomponent fibers 5 composing the fiber group 2 are melted through the heating step.

Alternatively, the above-described heating treatment may be executed at a step before the affixing of the fibers to the ends of the applicator bar 1 and subsequently, the affixing of the fibers to the bar ends may be executed.

According to the above-described applicator, the heat bondable bicomponent fibers 5 in the fiber group 2 are melted to be stuck to one another, whereby all the fibers composing the fiber group 2 are interwined to be prevented from being frayed. Accordingly, when water adheres to the fiber group 2 or when the fiber group 2 is immersed in the water, each fiber composing the fiber group 2 does not lose its combining force nor is not frayed. Consequently, the water proof and endurability of the applicator may be remarkably improved.

When the temperature of heat and the heating period at the step of heating the fiber group 2 are varied in accordance with the kinds of first and second components composing the heat bondable bicomponent fiber 5 or the ratio of the capacity of the first component to that of the first component, an amount of the meltable layer 4 melted by way of heating treatment may be changed relative to the whole amount thereof. Accordingly, since the touch feeling and the hardness of the fiber group 2 of the applicator are changed, such a heating step as described above also serves to control the quality of the applicators in accordance with the purposes of its use.

Furthermore the softness and the water contentability of the fiber group 2 may be controlled by changing the mixing ratio of the heat bondable bicomponent fibers 5 and the other natural fibers both composing the fiber group 2. The fiber group 2 may be composed only of the heat bondable bicomponent fibers 5.

Fig. 4 illustrates the applicator of a second embodiment. The applicator of the second embodiment differs from that of the foregoing embodiment in that the capacity of the fiber group 2 is sufficiently increased so that the applicator is especially suitable for artificial insemination of fruit trees and in that the fiber group 2 is affixed to either one of ends of the bar 1.

The foregoing disclosure and drawings are merely illustrative of the principles of the present invention and are not to be interpreted in a limiting sense. The only limitation is to be determined from the scope of the appended claims.

## Claims

1. An applicator comprising a bar (1) having two ends and a cotton-form group of fibers (2) affixed to either of the ends of the bar (1), characterized in that the group of fibers (2) including heat bondable bicomponent fibers each having a meltable layer (4) on the surface thereof, said heat bondable bicomponent fibers being combined with one another by way of heating.

2. An applicator as claimed in claim 1, wherein each heat bondable bicomponent fiber includes a first component consisting of a polymer having fiber-forming property and a second component consisting of a polymer having fiber-forming property and a melting point at least 20°C lower than that of the first component and wherein the first and second components are combined with each other in either a parallel or sheathed arrangement so that a ratio of an arc portion of the cross sectional circumference of the second component to the cross sectional circumference of the first component takes the value ranging from 50% to 100%.

3. An applicator as claimed in claim 2, wherein said group of fibers (2) includes at leat 10 weight percentage of the heat bondable bicomponent fibers and either natural or synthetic fibers.

4. A method of forming an applicator comprising steps of:

a) forming a cotton-form group of fibers (2) by affixing fibers including heat bondable bicomponent fibers to either of both ends of a bar (1); and

b) heat-treating said group of fibers (2) so that the heat bondable bicomponent fibers are combined with one another.

5. A method of forming an applicator as claimed in claim 4, wherein each heat bondable bicomponent fiber includes a first component consisting of a polymer having fiber-forming property and a second component consisting of a polymer having fiber-forming property and a melting point at least 20°C lower than that of the first component and wherein the first and second components are combined with each other in either a parallel or sheathed arrangement so that a ratio of an arc portion of the cross sectional circumference of the second component to the cross sectional circumference of the first component takes the value ranging from 50% to 100%.

6. A method of forming an applicator as claimed in claim 5, wherein said group of fibers (2) consists of at least 10 weight percentage of the heat bondable bicomponent fibers and either natural or synthetic fibers.

7. A method of forming an applicator comprising steps of:

a) combining heat bondable bicomponent fibers each having a meltable layer (4) on the surface thereof with one another or with other fibers into a cotton-form;

b) heat-treating said cotton-form heat bondable bicomponent fibers so that the cotton-form heat bondable bicomponent fibers are combined with one another; and

c) affixing said heat bondable bicomponent fibers to either one or both ends of a bar (1) so that a group of fibers (2) is provided thereon.

Fig.1

Fig.2   Fig.3   Fig.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 846 593 (LARSSON) * Figures; page 18, lines 14-22; page 24, lines 2-17; page 25, lines 11-25 * | 1-3 | A 61 F 13/20 |
| Y | | 4-7 | |
| Y | EP-A-0 089 271 (TRAN DINH) * Abstract; page 5, lines 29-36; page 6, lines 14-18 * | 4-7 | |
| A | FR-A-2 579 098 (TRAN DINH) | | |
| A | US-A-3 586 380 (ALIBECKOFF) | | |
| A | DE-U-8 533 322 (HARTMANN) | | |

TECHNICAL FIELDS
SEARCHED (Int. Cl.5)

A 61 F

The present search report has been drawn up for all claims

| Place of search | Date of completion of the search | Examiner |
|---|---|---|
| THE HAGUE | 01-08-1989 | STEENBAKKER J. |